# DEMANDE DE BREVET EUROPEEN

(11) **EP 1 591 134 A1**
(43) Date de publication de la demande: **02.11.2005**
(21) Numéro de dépôt: 05290879.5
(22) Date de dépôt: 20.04.2005
(51) Int. Cl.: A61L 31/12

(54) **Implant textile de réfection pariétale**

(30) Priorité: 21.04.2004 FR 0404193
(71) Demandeur: Cousin Biotech, 59117 Wervicq Sud (FR)
(72) Inventeur: Leroy, Joel, 62160 Bully Les Mines (FR); Marescaux, Jacques, 67310 Scharrachbergheim (FR); Mutter, Didier, 67550 Vendenheim (FR); Vix, Michel, 67207 Niederhausbergen (FR)
(74) Mandataire: Thinat, Michel

(57) **Abrégé**

L'invention concerne un implant chirurgical textile biocompatible partiellement biodégradable par l'organisme, composé de deux composants synthétiques, l'un résorbable, l'autre non résorbable.

Selon l'invention, la structure non résorbable, dotée d'un faible allongement en traction, est associée à une structure résorbable composée d'un matériau bioactif favorisant l'apparition d'une fibrose organisée.

L'implant de l'invention s'applique notamment à la réfection chirurgicale prothétique pariétale, notamment en chirurgie de réfection abdominale.

## Description

La présente invention se rapporte au domaine technique des implants prothétiques chirurgicaux textiles de réfection pariétale, utiles en chirurgie viscérale et plus particulièrement en chirurgie herniaire, pour la réfection de la paroi abdominale, mais pouvant s'appliquer dans d'autres indications chirurgicales.

Les implants textiles pour le traitement des hernies de la paroi abdominale sont connus depuis plusieurs décennies. La première publication à ce sujet, où l'utilisation d'un implant tricoté de fils de polypropylène fut décrite, remonte à l'année 1963 (Usher FC. "Hernia repair with knitted polypropylene mesh" Surg. Gynecol. Obstet. 1963 Aug; 117:239-40). L'utilisation de ces implants en fils de polypropylène fut popularisée par Lichtenstein, grâce à la description de sa technique de réparation des hernies inguinales en 1986. D'autres implants, en matériaux résorbables ou non, comme le polyester, l'Acide Polylactique et même des produits à base de fils métalliques, furent proposés (Adloff M., Arnaud JP. "Experimental study of résistance and biologic tolerance of prosthetic materials used in the repair of losses of substance of the abdominal wall". Chirurgie. 1976 Jun; 102 (6) :390-6) .

De nos jours, les différents implants textiles de réfection pariétale commercialisés répondent à des critères de biocompatibilité, d'allongement à la traction et de résistance à l'infection.

Le polypropylène reste un composant de référence, notamment sous sa forme de monofilament tricoté, objet de différents implants dont le Prolene® de la société Ethicon.

Ces produits tricotés qui ont prouvé leur efficacité présentent des inconvénients. En effet le poids élevé de ces implants compris entre 80 et 100 gr/m² confère une certaine rigidité à l'implant, utile pour son placement. Cependant, la quantité de matière trop importante peut provoquer des intolérances.

Il existe également des prothèses de hernie en monofilament polyamide tissé, type plaques Kron® commercialisées par la société B. Braun. Ces produits sont encore plus rigides. Ils ont le désavantage de comporter une masse de matière implantée importante. Ces caractéristiques, en particulier de rigidité permanente, sont parfois une gène pour le patient.

De plus, les prothèses non résorbables à base de fils de polypropylène tricotés s'accompagnent d'une réaction inflammatoire humorale et cellulaire importante, source d'une fibrose inorganisée avec dépôt de collagène, qui en cas d'excès, entraîne un rétrécissement de la prothèse implantée jusqu'à 20% de sa surface, voire jusqu'à 60% de son volume, pour certains obturateurs type Plug.

Des travaux expérimentaux réalisés sur le rat Wistar, à l'Institut de Recherche contre les Cancers de l'Appareil Digestif (I.R.C.A.D., Strasbourg) par le Docteur Tanaka et al. sous la direction du Professeur Mutter (MD, PhD) et du Professeur Leroy (MD) , qui seront présentés au Congrès du 29, 30 avril et 1er mai 2004 de l'European Hernia Society de Prague, confirment ces phénomènes biologiques. Ces études montrent que des implants à base de polypropylène type Prolene® et à base de polypropylène allégé entraînent rapidement une réaction inflammatoire cellulaire importante qui est suivie d'une réaction de fibrose non organisée, puis d'une fixation de collagène.

Ainsi une prothèse de 100g/m² s'accompagnera d'une même réaction inflammatoire qu'une prothèse de 30g/m². La réaction fibreuse inorganisée apparaît tardivement à partir du 15ème jour. Cette apparition tardive explique des phénomènes de rétrécissement de la structure de l'implant décrits sous le nom de « shrinkage » par les auteurs anglo-saxons. Cette réaction de rétrécissement est plus marquée pour l'implant le plus allégé. La réaction de rétrécissement (shrinkage) apparaît rapidement dans ce modèle expérimental, quel que soit le type de prothèse de polypropylène, ce qui est un obstacle majeur à la bonne intégration de l'implant.

L'association de matériaux résorbables entraînant une augmentation de la rigidité initiale de ce type d'implant a été proposée, dans l'objectif de faciliter la pause. Cependant, les travaux conduits par Schumpelick et présentés lors du congrès de la « European Hernia Society » à Londres en 2003, faisaient état d'un taux de récidive au moins deux fois plus important que la moyenne avec ce type d'implant, ceci en raison de problèmes techniques dus à l'implantation.

La présente invention a pour objet de supprimer les différents inconvénients cités grâce à un implant en textile synthétique biocompatible à long terme constitué d'une structure de base en matériau non résorbable et d'une structure secondaire en matériau résorbable. Quelque soit l'effort engagé, l'allongement à la traction de la structure de base non résorbable de l'implant est inférieur à l'allongement des tissus du site d'implantation. La structure secondaire de l'implant en matériaux résorbables permet de favoriser l'apparition d'une fibrose organisée, dans les quinze premiers jours après implantation. Cette fibrose a pour but quant à elle d'éviter le rétrécissement futur de l'implant après sa pose.

L'implant peut être un tricot, un tissé, un tissé multiaxial ou une tresse.

La masse initiale de l'implant selon l'invention, avant implantation et résorption, rappelle celle des implants couramment utilisés qui ont un poids de l'ordre de 100g/m².

Le matériau résorbable de l'invention est qualifié de bioactif puisqu'il entraîne l'apparition d'une fibrose organisée rapide dans les 15 premiers jours après implantation, le plus souvent parallèle aux fibres de matériaux synthétiques non résorbables, par exemple en polypropylène. Cette fibrose précède une réaction inflammatoire cellulaire qui est majeure à 15 jours et jusqu'à huit semaines. La réaction inflammatoire est au moins 50 % inférieure à la réaction inflammatoire de la structure de base, polypropylène seul, allégé ou non, et ceci, sans gêner l'apparition du collagène qui est également parfaitement organisée. Ce comportement biologique a pu être vérifié lors de travaux expérimentaux réalisés à I'I.R.C.A.D par le Dr Tanaka et al (précités).

Les dessins annexés illustrent l'invention :

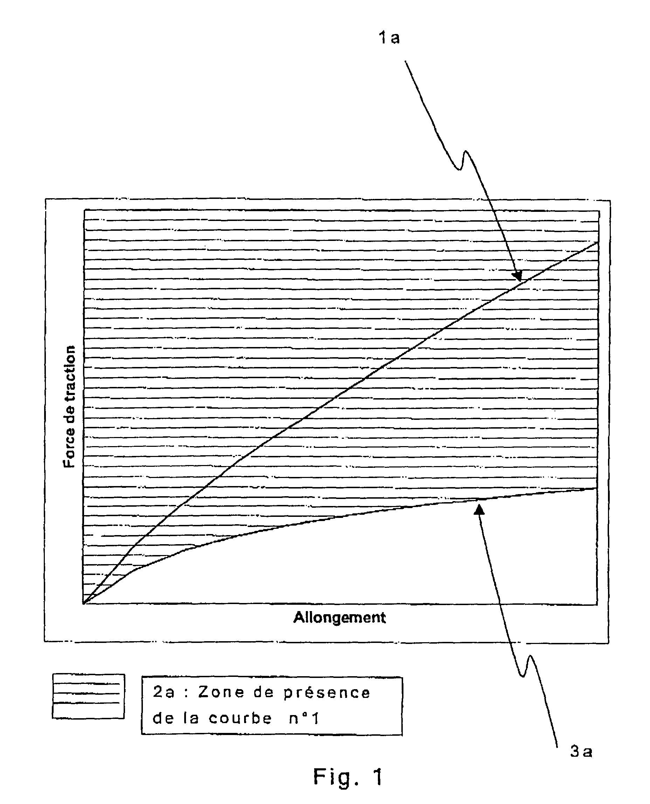

La figure 1 est un graphique représentant la résistance de l'implant à la traction.
La figure 2 représente l'implant réalisé sous la forme d'un tissé multiaxial.
En référence à ces dessins, la courbe de la figure 1 montre une zone hachurée (2a) dans laquelle se trouve la courbe de traction (1a) de la structure non résorbable selon l'invention. La courbe (3a) caractérise le comportement mécanique des tissus du site d'implantation.
Dans un mode préféré de réalisation, la structure non résorbable est réalisée en polypropylène, en polyester, en polyamide ou en polyéthylène et le matériau résorbable de la structure secondaire est en acide poly L lactique (PLLA).

La masse de la structure non résorbable est de 10 à 80 pour cent de la masse totale de l'implant. De préférence l'implant a une masse de produit non résorbable de 30 à 40 pour cent de sa masse totale.

Il existe de nombreuses techniques textiles pour obtenir un implant correspondant à l'invention, données à titre d'exemples non limitatifs.

Dans un mode préféré de réalisation, on réalise un textile tissé ou un textile tricoté avec un fil composite constitué d'une matière résorbable et d'une matière non résorbable. La matière non résorbable peut-être un polypropylène. La matière résorbable peut-être un Acide Poly L Lactique (PLLA).

Le fil composite peut être :
- un fil de 150 dtex réalisé par torsion de 300 tours par mètre, de fibres ou filaments de 5 dtex en polypropylène et de fibres ou filaments de 5 dtex en PLLA.
- un fil retord de 150 dtex. Ce fil est réalisé par torsion de deux fils simples constitués de fibres ou de filaments en polypropylène et de fibres ou de filaments en PLLA. Les fibres ou les filaments de chaque fil simple sont assemblés aussi par torsion.
- un fil guipé de 150 dtex avec un fil non résorbable en âme de 50 dtex en polypropylène et d'une couverture résorbable en fibres 5 dtex en PLLA.
- un fil guipé constitué d'un fil en âme de 150 dtex en polypropylène et d'un fil résorbable de 50 dtex enroulé autour de l'âme.
- un fil coextrudé. Le polymère PLLA en fusion est enduit à l'aide d'une extrudeuse autour d'une âme constituée d'au moins un fil mono filament ou multi filament polypropylène de 150 dtex.
- une tresse composée d'un fil en âme de 50 dtex en polypropylène et d'une couverture 4 fuseaux en fil PLLA de 50dtex.
- une structure composée d'un fil en âme de 50 dtex en polypropylène dont la couverture, tricotée en mailles cueillies ou jetées, est en PLLA.
- un trempage d'un fil polypropylène 50 dtex enduit par une solution de PLLA dans du chloroforme. La solution comporte du PLLA d'indice de viscosité 3.07 dl/g déterminé selon la norme ISO 1628-1, dans du chloroforme (solvant).

Les concentrations peuvent être comprises entre 10 et 30 gr de PLLA pour 100 cm3 de chloroforme, particulièrement 15 gr pour 100cm³.

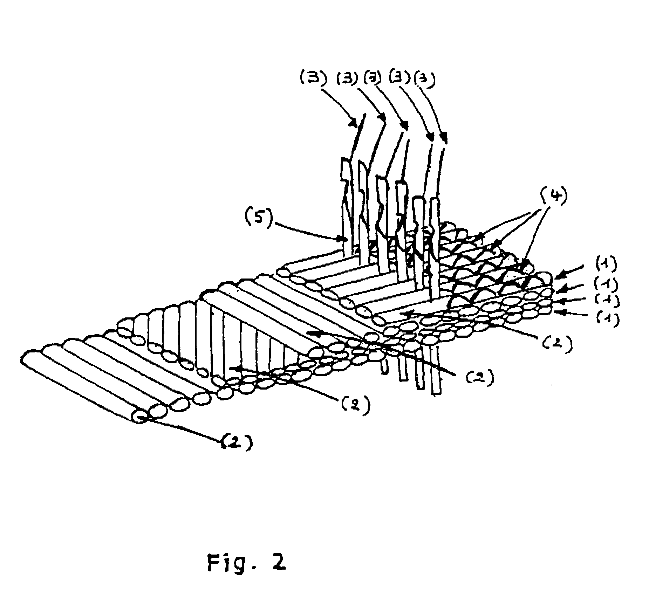

Dans une forme particulière de réalisation, l'implant est obtenu par la technique du tissage multiaxial, représenté en figure 2. Plusieurs couches de surfaces textiles (1) sont assemblées par couture (4) afin d'obtenir un textile dont les fils composites (2) sont orientés dans de multiples directions de l'espace. Les couches de surfaces textiles sont cousues à l'aide d'aiguille (5) entraînant un fil à coudre (3). Le fil composite (2) peut être un mélange de fibres non résorbables et de fibres résorbables assemblées par torsion ou retordage. Le fil composite (2) peut aussi prendre la forme d'un fil guipé avec une âme non résorbable et une couverture de fibres résorbables. L'âme du fil guipé peut-être un fil multifilament ou un filé de fibres polypropylène, la couverture du fil guipé peut-être constituée de fibres d'Acide Poly L Lactique (PLLA).

L'invention peut être réalisée par imprégnation. On réalise une solution de matière résorbable dans un solvant puis on imprègne par trempage une structure non résorbable en tissé multiaxial, tissée, tricotée, tressée ou non tissée. La matière non résorbable peut-être un polypropylène, la solution de matière résorbable peut-être un acide poly L lactique d'indice de viscosité 3.07 dl/g, déterminé selon la norme ISO 1628-1 dans du chloroforme (solvant). Les concentrations peuvent être comprises entre 1 et 30 g, notamment 5 g, de PLLA pour 100 cm³ de chloroforme.

L'invention peut être réalisée par mélange intime de fibres ou de filaments non résorbables avec des fibres ou des filaments résorbables. Le matériau non résorbable peut-être un polypropylène, le matériau résorbable peut-être du PLLA. Les fibres ou les filaments peuvent être intimement mélangées par une première opération de cardage et d'aiguilletage. On obtient ainsi une nappe. Cette nappe passe ensuite dans une calandre dont l'un des rouleaux est chauffant. Cette dernière opération de fusion thermique permet de lier les fibres ou les filaments entre eux.

L'invention peut être réalisée par tissage, par tissage multiaxial ou par tricotage d'au moins un fil non résorbable et d'au moins un fil résorbable. Il est par exemple possible de tisser une toile ou un sergé comportant un fil sur deux résorbable et un fil sur deux non résorbable que ce soit dans le sens chaîne ou dans le sens trame. Le fil non résorbable peut-être un polypropylène, le fil résorbable peut-être en PLLA.

En cas d'implant de petite largeur, la technique du tressage plat ou du tressage tubulaire ouvert ensuite en long peut être envisagée.

L'implant peut-être imprégné de matière médicamenteuse active. Par exemple de l'héparine qui a une action anti-coagulante ou un antibiotique qui a une action anti-infectieuse.

L'application décrite correspond à la fabrication d'un implant pour la réparation d'une hernie, mais l'invention peut s'appliquer aux autres domaines de la chirurgie viscérale, tels que: incisions chirurgicales viscérales et pariétales, hernies diaphragmatiques, éventrations, traitement de l'incontinence urinaire et du prolapsus en uro-gynécologie, réparation des tendons, ligaments et coiffes articulaires en orthopédie, des parois vasculaires, des enveloppes neurologiques (méninges, nerfs).

En outre, et bien que la présente invention ait été décrite en référence à des modes de réalisation préférés, diverses modifications connues des hommes de l'art peuvent être faites aux structures présentées ci-dessus sans s'écarter de l'invention telle que revendiquée.

## Revendications

1. Implant en textile synthétique biocompatible **caractérisé en ce qu'**il comprend :
- une structure de base, en matériau non résorbable, dotée quelque soient les efforts engagés d'un allongement à la traction inférieur à l'allongement à la traction des tissus du site d'implantation.
- une structure secondaire en matériau résorbable.

2. Implant selon la revendication 1, **caractérisé en ce que** le matériau résorbable utilisé est de l'Acide Poly L Lactique qui entraîne l'apparition d'une fibrose organisée dans les 15 premiers jours après implantation, et une réaction inflammatoire faible au moins inférieure à 50% à la réaction inflammatoire du matériau de la structure non résorbable seul.

3. Implant selon l'une des revendications 1 à 2, **caractérisé en ce qu'**il est un tricot, un tissé, un tissé multiaxial ou une tresse.

4. Implant selon la revendication 3, **caractérisé en ce que** le tricot, le tissé, le tissé multiaxial ou la tresse est réalisé avec au moins un fil composite, constitué de matériaux résorbables en couverture et de matériaux non résorbables en âme.

5. Implant selon la revendication 4, **caractérisé en ce que** le fil composite est réalisé selon les techniques suivantes : guipage, trempage, co-extrusion, tressage ou tricotage tubulaire.

6. Implant selon la revendication 3, **caractérisé en ce qu'**il est réalisé à partir d'au moins un fil composite obtenu par mélange de fibres non résorbable et de fibres résorbables assemblées par torsion ou retordage.

7. Implant selon la revendication 3, **caractérisé en ce qu'**il est un tricot, un tissé, un tissé multiaxial, un tressé ou un non tissé, en matériau non résorbable, imprégné ou trempé dans une solution de matériau résorbable.

8. Implant selon l'une des revendications 1 à 2, **caractérisé en ce qu'**il est un non tissé comportant un mélange intime de fibres ou filaments non résorbables et de fibres ou filaments résorbables assemblées par cardage/aiguilletage puis calandrage.

9. Implant selon l'une des revendications 1 à 2, **caractérisé en ce qu'**il est un tissu , un tissu multiaxial un tricot réalisé avec au moins un fil résorbable et au moins un fil non résorbable.

10. Implant selon l'une quelconque des revendications précédentes **caractérisé en ce qu'**il contient une matière complémentaire médicamenteuse active.

11. Implant selon l'une quelconque des revendications précédentes, **caractérisé en ce que** le composant non résorbable est un Polypropylène, un Polyester, un Polyamide, un Polyéthylène.

12. Implant selon l'une quelconque des revendications précédentes, **caractérisé en ce que** la masse du composant non résorbable est de 10 à 80 pour cent de la masse totale de l'implant.
